# EUROPEAN PATENT APPLICATION

(11) **EP 4 119 674 A1**
(43) Date of publication of application: **18.01.2023**
(21) Application number: 21793623.6
(22) Date of filing: 23.03.2021
(51) Int. Cl.: C12Q 1/02, C12M 1/42, C12M 3/00, C12N 5/02, C12N 13/00, C12P 21/08, G06N 20/00

(54) **METHOD FOR ESTIMATING CULTURE STATE, INFORMATION PROCESSING DEVICE, AND PROGRAM**

(30) Priority: 21.04.2020 JP 2020075480
(71) Applicant: FUJIFILM Corporation, Tokyo 106-8620 (JP)
(72) Inventor: NAKAMURA, Naoki, Ashigarakami-gun, Kanagawa 258-8577 (JP); HARAGUCHI, Nobuyuki, Ashigarakami-gun, Kanagawa 258-8577 (JP)
(74) Representative: Hoffmann Eitle
(86) International application number: PCT/JP2021/012016
(87) International publication number: WO 2021/215179

(57) **Abstract**

Spectral data indicating an intensity of electromagnetic waves, which have been emitted to a cell suspension including a cell and a culture solution and have been subjected to an action of the cell suspension, for each wave number or wavelength is acquired. Preprocessing is performed on the spectral data. A soft sensor, which receives processed data obtained by the preprocessing as an input and outputs state data indicating a state of the cell or the culture solution, is constructed by machine learning using a plurality of combinations of the processed data and the state data as training data. The processed data for the spectral data acquired for a cell suspension including a cell which is being cultured is input to the soft sensor, and the state data output from the soft sensor is acquired.

## Description

### BACKGROUND OF THE INVENTION

### 1. Field of the Invention

The technology of the present disclosure relates to a method for estimating a culture state, an information processing device, and a program.

### 2. Description of the Related Art

The following techniques are known as techniques related to a culture state estimation method that estimates the state of a cells which are being cultured or a culture solution. For example, JP2019-110767A discloses a method for controlling a cell culture device comprising a culture tank in which a culture solution is enclosed and cells are cultured to produce a target substance, a measurement unit that measures an operating state of the culture tank, an individual control unit that individually controls various ventilation gas supply units and the like such that a measured value by the measurement unit is equal to a preset control target value, and a soft sensor that is connected to the individual control unit such that it can bidirectionally communicate with the individual control unit and has a pre-constructed statistical numerical calculation model. After individual control values by the individual control unit are input, the soft sensor estimates the quality properties of the target substance in the culture tank using the control values and the statistical numerical calculation model and corrects the control target values in a case in which the estimated quality properties are less than a preset reference value.

Further, JP2008-526203A discloses a bioreactor comprising a cell growth vessel and a sensor. The sensor is configured to measure the internal state of the vessel and to provide an input to a model-free adaptive controller and measures a state indicating a correlation with a product quality attribute using a method such as spectrophotometry. The adaptive controller is configured to include a dynamic feedback system using an artificial neural network and to provide an output to an actuator.

### SUMMARY OF THE INVENTION

A culture state for cell culture period is monitored by actually measuring a predetermined measurement item for some cells or the culture solution sampled from the culture vessel. However, this offline process requires preprocessing for a sample, and it takes a relatively long time to obtain a monitoring result. Therefore, in a case in which any abnormality occurs for the culture period, it is difficult to respond to the case immediately.

In addition, it is considered that spectroscopy is used as a method for monitoring the culture state. In the monitoring of the culture state using spectroscopy, a cell suspension including cells and a culture solution is irradiated with electromagnetic waves, and spectral data indicating the intensity of the electromagnetic waves, which have been subjected to the action of the cell suspension, for each wave number or wavelength is analyzed by a multivariate analysis method, such as partial least squares regression (PLS), to acquire an estimated value related to an object (for example, an antibody produced from a cell) to be monitored. However, in an actual culture process in which the density of cells in the culture vessel is high (that is, there are many impurities) and the content of the object to be monitored is small, in a case in which the culture state is estimated using a multivariate analysis method, such as PLS, the accuracy of estimation may be significantly reduced.

The technology of the present disclosure has been made in view of the above-mentioned problems, and an object of the technology of the present disclosure is to provide a method for estimating a culture state, an information processing device, and a program that can obtain an estimation result immediately and can be applied even in a case in which the density of cells in a culture vessel is high and the content of an object to be monitored is low.

According to the technology of the present disclosure, there is provided a method for estimating a culture state. The method comprises: acquiring spectral data indicating an intensity of electromagnetic waves, which have been emitted to a cell suspension that includes a cell and a culture solution and has a cell concentration of 35 × 106 cells/mL or more and have been subjected to an action of the cell suspension, for each wave number or wavelength; constructing a soft sensor, which receives the spectral data as an input and outputs state data indicating a state of the cell or the culture solution, with machine learning using a plurality of combinations of the spectral data and the state data as training data; and inputting the spectral data acquired for a cell suspension including a cell which is being cultured to the soft sensor and acquiring the state data output from the soft sensor.

In addition, according to the technology of the present disclosure, there is provided another method for estimating a culture state. The method comprises: acquiring spectral data indicating an intensity of electromagnetic waves, which have been emitted to a cell suspension including a cell and a culture solution and have been subjected to an action of the cell suspension, for each wave number or wavelength; performing preprocessing on the spectral data; constructing a soft sensor, which receives processed data obtained by the preprocessing as an input and outputs state data indicating a state of the cell or the culture solution, with machine learning using a plurality of combinations of the processed data and the state data as training data; and inputting the processed data for the spectral data acquired for a cell suspension including a cell which is being cultured to the soft sensor and acquiring the state data output from the soft sensor.

The preprocessing may include a process of selecting, from spectral intensity values for each wave number or wavelength included in the spectral data, a spectral intensity value used as the training data. Preferably, among the spectral intensity values for each wave number or wavelength included in the spectral data, the number of spectral intensity values selected to be used as the training data is equal to or greater than 5 and less than 1000. The selection may be performed using sparse modeling. The preprocessing may include specifying high-correlation spectral data having a relatively high correlation with the state data among the spectral data as the processed data.

The spectral data may be data indicating a spectrum of scattered light of light emitted to the cell suspension.

The state data may be data related to an amount of antibody included in the culture solution, an amount of decomposition product of the antibody, an amount of aggregate of the antibody, or an amount of immature sugar chain having a structure similar to that of the antibody. In addition, the state data may be data related to an amount of component included in the culture solution. Further, the state data may be data related to the number of cells.

According to the technology of the present disclosure, there is provided an information processing device comprising at least one processor. The processor performs preprocessing on spectral data indicating an intensity of electromagnetic waves, which have been emitted to a cell suspension including a cell and a culture solution and have been subjected to an action of the cell suspension, for each wave number or wavelength and constructs a soft sensor, which receives processed data obtained by the preprocessing as an input and outputs state data indicating a state of the cell or the culture solution, with machine learning using a plurality of combinations of the processed data and the state data as training data.

According to the technology of the present disclosure, a program that causes a computer to execute a process comprising: performing preprocessing on spectral data indicating an intensity of electromagnetic waves, which have been emitted to a cell suspension including a cell and a culture solution and have been subjected to an action of the cell suspension, for each wave number or wavelength; and constructing a soft sensor, which receives processed data obtained by the preprocessing as an input and outputs state data indicating a state of the cell or the culture solution, with machine learning using a plurality of combinations of the processed data and the state data as training data.

The technology of the present disclosure provides a method for estimating a culture state and an information processing device that can obtain an estimation result immediately and can be applied even in a case in which the density of cells in a culture vessel is high and the content of an object to be monitored is low.

### BRIEF DESCRIPTION OF THE DRAWINGS

Fig. 1 is a diagram illustrating an example of a method for estimating a culture state according to an embodiment of the technology of the present disclosure.
Fig. 2 is a diagram illustrating an example of a method for acquiring spectral data.
Fig. 3 is a diagram illustrating an example of training data according to the embodiment of the technology of the present disclosure.
Fig. 4 is a diagram illustrating an example of the method for estimating the culture state according to the embodiment of the technology of the present disclosure.
Fig. 5 is a diagram illustrating an example of a hardware configuration of an information processing device according to the embodiment of the technology of the present disclosure.
Fig. 6 is a diagram illustrating an example of a structure of an estimation model according to the embodiment of the technology of the present disclosure.
Fig. 7 is an example of a functional block diagram illustrating an example of a functional configuration of the information processing device in a learning phase according to the embodiment of the technology of the present disclosure.
Fig. 8 is a flowchart illustrating an example of a flow of a soft sensor construction process according to the embodiment of the technology of the present disclosure.
Fig. 9 is an example of a functional block diagram illustrating an example of a functional configuration of the information processing device in an operation phase according to the embodiment of the technology of the present disclosure.
Fig. 10 is a flowchart illustrating an example of a flow of an estimation process according to the embodiment of the technology of the present disclosure.
Fig. 11 is a diagram illustrating an example of state data that can be acquired by a soft sensor.
Fig. 12A is a graph illustrating a relationship between an estimated value acquired by the soft sensor according to the embodiment of the technology of the present disclosure and a measured value acquired by sampling.
Fig. 12B is a graph illustrating the relationship between the estimated value acquired by the soft sensor according to the embodiment of the technology of the present disclosure and the measured value acquired by sampling.
Fig. 12C is a graph illustrating the relationship between the estimated value acquired by the soft sensor according to the embodiment of the technology of the present disclosure and the measured value acquired by sampling.
Fig. 12D is a graph illustrating the relationship between the estimated value acquired by the soft sensor according to the embodiment of the technology of the present disclosure and the measured value acquired by sampling.
Fig. 12E is a graph illustrating the relationship between the estimated value acquired by the soft sensor according to the embodiment of the technology of the present disclosure and the measured value acquired by sampling.
Fig. 12F is a graph illustrating the relationship between the estimated value acquired by the soft sensor according to the embodiment of the technology of the present disclosure and the measured value acquired by sampling.
Fig. 12G is a graph illustrating the relationship between the estimated value acquired by the soft sensor according to the embodiment of the technology of the present disclosure and the measured value acquired by sampling.
Fig. 12H is a graph illustrating a relationship between an estimated value acquired by a soft sensor according to an embodiment of the technology of the present disclosure and a measured value acquired by sampling.
Fig. 121 is a graph illustrating the relationship between the estimated value acquired by the soft sensor according to the embodiment of the technology of the present disclosure and the measured value acquired by sampling.

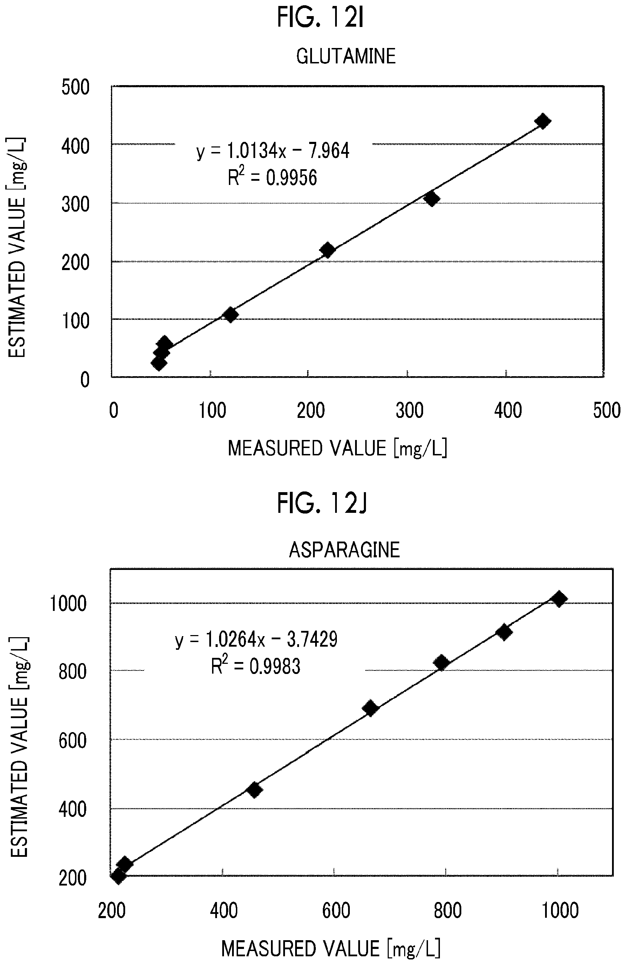

Fig. 12J is a graph illustrating the relationship between the estimated value acquired by the soft sensor according to the embodiment of the technology of the present disclosure and the measured value acquired by sampling.

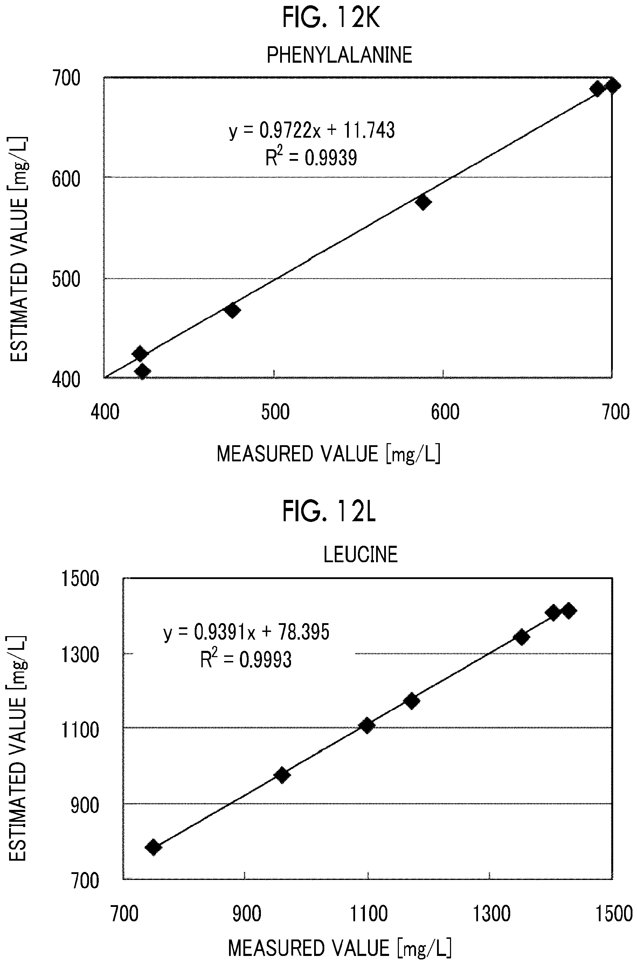

Fig. 12K is a graph illustrating the relationship between the estimated value acquired by the soft sensor according to the embodiment of the technology of the present disclosure and the measured value acquired by sampling.
Fig. 12L is a graph illustrating the relationship between the estimated value acquired by the soft sensor according to the embodiment of the technology of the present disclosure and the measured value acquired by sampling.

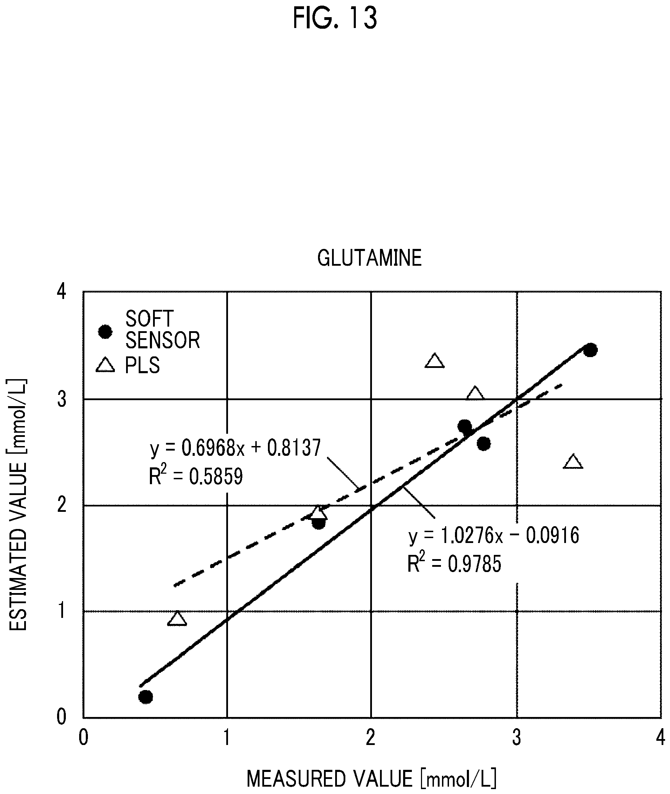

Fig. 13 is a graph illustrating a relationship between an estimated value of the concentration of glutamine included in a culture solution and a measured value in a case in which the estimated value is acquired by the soft sensor and in a case in which the estimated value is acquired by a multivariate analysis method using PLS according to the related art.

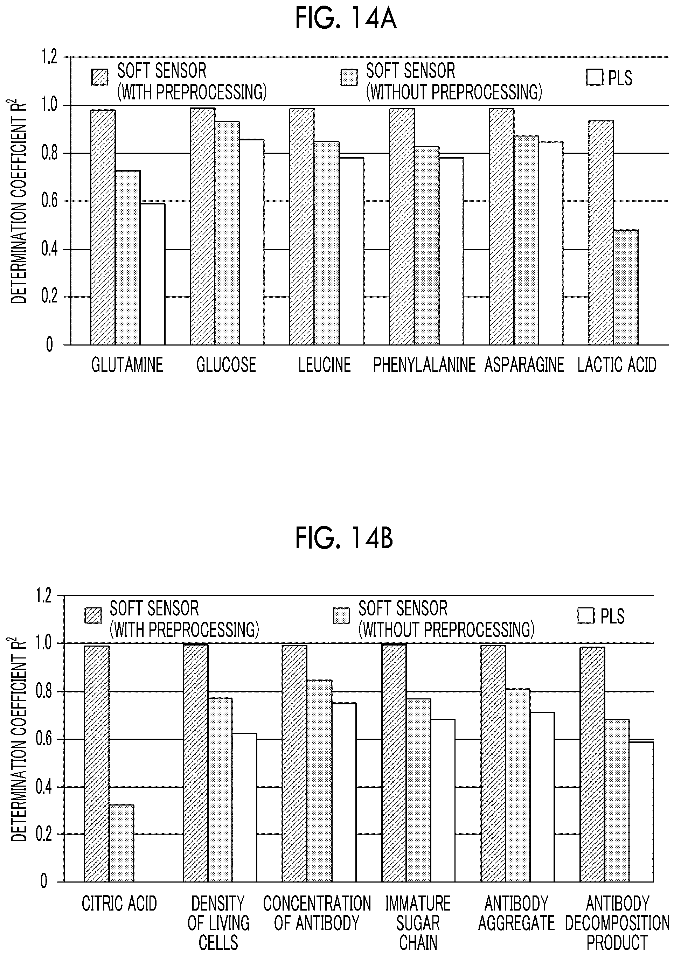

Fig. 14A is a graph illustrating determination coefficients in a case in which an estimated value of the concentration of each component included in the culture solution is acquired by the soft sensor and in a case in which the estimated value is acquired by the multivariate analysis method using PLS according to the related art.
Fig. 14B is a graph illustrating the determination coefficients in a case in which the estimated value of the concentration of each component included in the culture solution is acquired by the soft sensor and in a case in which the estimated value is acquired by the multivariate analysis method using PLS according to the related art.

### DESCRIPTION OF THE PREFERRED EMBODIMENTS

Hereinafter, an example of an embodiment of the technology of the present disclosure will be described with reference to the drawings. In addition, in each of the drawings, the same or equivalent components and portions are denoted by the same reference numerals, and the repeated description thereof will be appropriately omitted.

A method for estimating a culture state according to an embodiment of the technology of the present disclosure includes: a step of acquiring spectral data indicating the intensity of electromagnetic waves, which have been emitted to a cell suspension including a cell and a culture solution and have been subjected to the action of the cell suspension, for each wave number or wavelength; a step of performing preprocessing on the acquired spectral data; and a step of constructing a soft sensor that receives processed data obtained by the preprocessing as an input and outputs state data indicating the state of the cell or the culture solution with machine learning using a plurality of combinations of the processed data and the state data as training data. Dimensional reduction methods, such as sparse modeling, principal component analysis (PCA), latent semantic analysis (singular value decomposition) (LSA (SVD)), linear discriminant analysis (LDA), independent component analysis (ICA), and partial least squares regression (PLS), are used as a preprocessing method. The preprocessing method may include a process of selecting a spectral intensity value used as the training data among the spectral intensity values for each wave number or wavelength included in the spectral data. In this case, the remaining spectral intensity values for each wave number or wavelength after the selection are the processed data. It is assumed that the spectral intensity values for each wave number or wavelength constituting the spectral data are enormous. The selection of the data used as the training data makes it possible to prevent a reduction in the accuracy of prediction caused by over-training with model data. The selection of the spectral data can be performed, for example, by sparse modeling. That is, the preprocessing performed on the spectral data may include a process of excluding data having a relatively low correlation with the state data among the spectral data, using sparse modeling, to specify high-correlation spectral data having a relatively high correlation with the state data among the spectral data as the processed data. Among the spectral intensity value for each wave number or wavelength included in the spectral data, the number of spectral intensity which are used as the training data and selected by the preprocessing is preferably equal to or greater than 5 and less than 1000, more preferably equal to or greater than 5 and equal to or less than 800, further preferably equal to or greater than 5 and equal to or less than 500, and most preferably equal to or greater than 11 and equal to or less than 334.

In this embodiment, the sparse modeling means that explanatory variables are selected (that is, some of the explanatory variables are excluded) for a regression model which uses the spectral intensity values for each wave number or wavelength included in the spectral data as the explanatory variables and uses the state data as objective variables. For example, lasso regression can be used as a sparse modeling method. The lasso regression is a method that selects the explanatory variables such that a cost function calculated by adding a penalty term to a root mean squared error (RMSE) is minimized. In this embodiment, the explanatory variables are selected by excluding low-correlation spectral data having a relatively low correlation with the state data among the spectral data. The penalty term may be determined by, for example, cross-validation represented by K-fold cross validation. In the following description, a case in which the preprocessing performed on the spectral data is a process of specifying high-correlation spectral data will be described as an example.

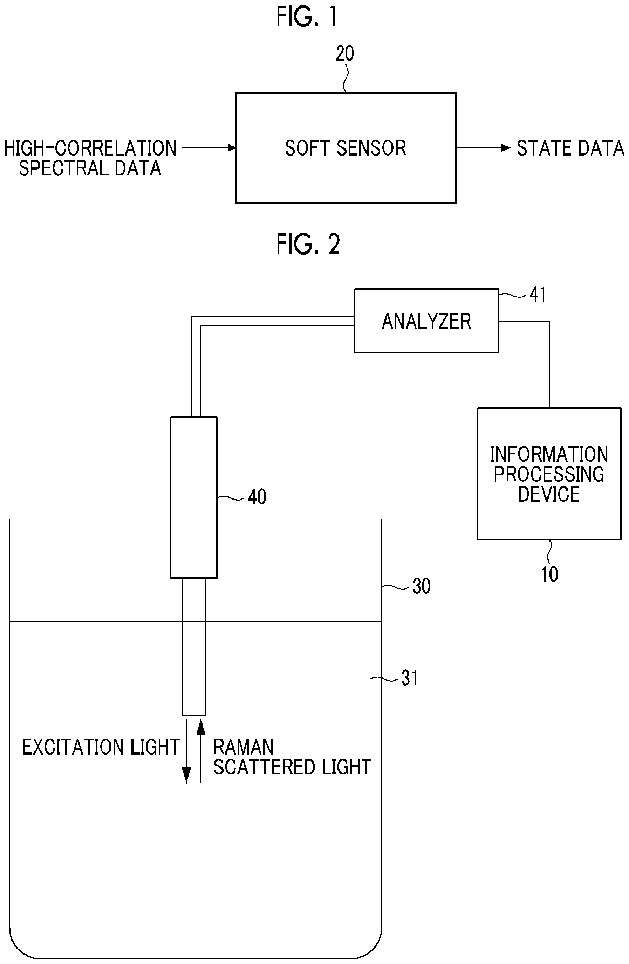

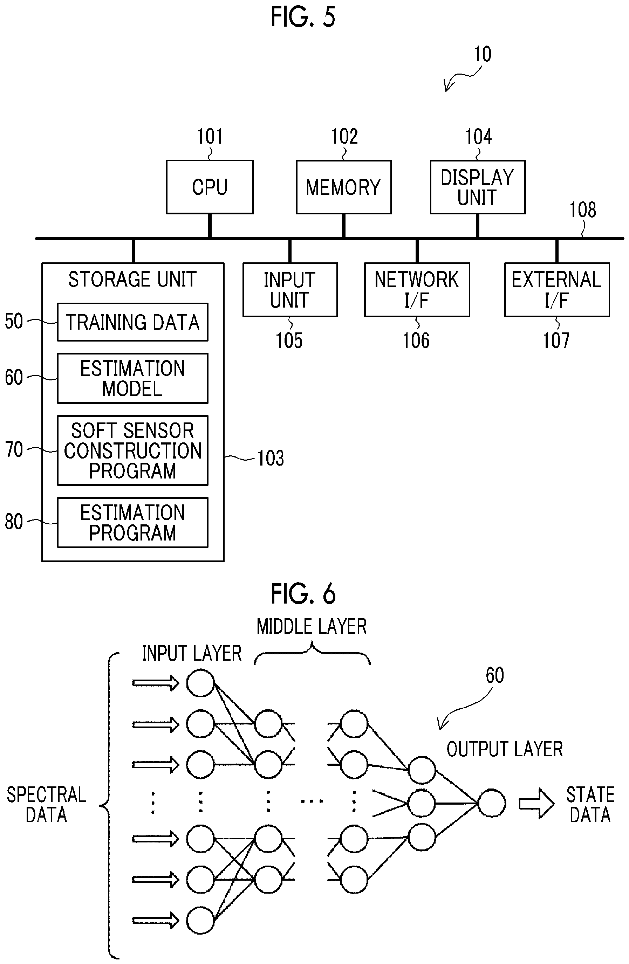

As illustrated in Fig. 1, the method for estimating the culture state according to the embodiment of the technology of the present disclosure includes a step of acquiring state data output from a soft sensor 20 in a case in which high-correlation spectral data is input as the processed data to the soft sensor 20 among the spectral data acquired for the cell suspension including the cells that are being cultured. The soft sensor 20 implements a process of outputting the state data on the basis of the input high-correlation spectral data using software. The soft sensor 20 is constructed in an information processing device 10 (see Figs. 2 and 5) which will be described below.

In this embodiment, an analysis method using Raman spectroscopy is applied to the soft sensor 20. That is, spectral data of Raman scattered light is applied as the spectral data input to the soft sensor 20. The Raman spectroscopy is a spectroscopic method that evaluates a substance using the Raman scattered light. In a case in which a substance is irradiated with light, the light interacts with the substance to generate Raman scattered light having a wavelength different from that of incident light. Since a difference in wavelength between the incident light and the Raman scattered light corresponds to the molecular vibration energy of the substance, the Raman scattered light having a different wavelength (wave number) can be obtained between substances having different molecular structures. In addition, it is possible to estimate various physical properties, such as stress, temperature, electrical characteristics, orientation, and crystallinity, using the Raman scattered light.

Fig. 2 is a diagram illustrating an example of a method for acquiring spectral data for a cell suspension 31 including the cells that are being cultured. The cells are cultured in a state in which the cells are accommodated in a culture vessel 30 together with a culture solution. The spectral data can be acquired using a known probe 40 and a known analyzer 41 for Raman spectroscopic analysis. As illustrated in Fig. 2, a tip of the probe 40 is immersed in the cell suspension 31 including the cells and the culture solution accommodated in the culture vessel 30. The cell suspension 31 is irradiated with excitation light emitted from a light emitting unit (not illustrated) that is provided at the tip of the probe 40. The Raman scattered light generated by the interaction between the excitation light and the cell suspension 31 is received by a light receiving unit (not illustrated) that is provided at the tip of the probe 40. The acquired Raman scattered light is decomposed for each wave number (the reciprocal of the wavelength) by the analyzer 41, and spectral data which is a spectral intensity value for each wave number is generated. In addition, the spectral data may be a spectral intensity value for each wavelength. In a case in which the spectral data is acquired by a perfusion culture method as the cell culture method, it is preferable to irradiate the cell suspension having a cell concentration equal to or greater than 35 × 10⁶ cells/mL with electromagnetic waves. The cell concentration is more preferably equal to or greater than 40 × 10⁶ cells/mL and equal to or less than 300 × 10⁶ cells/mL, further preferably equal to or greater than 60 × 10⁶ cells/mL and equal to or less than 220 × 10⁶ cells/mL, and most preferably equal to or greater than 80 × 10⁶ cells/mL and equal to or less than 1300 × 10⁶ cells/mL. In a case in which cells are cultured in order to produce an antibody, as the concentration of the cells to be cultured becomes higher, the efficiency of producing the antibody can become higher. Therefore, predetermined production efficiency can be ensured by setting the cell concentration to be equal to or greater than 35 × 10⁶ cells/mL. On the other hand, in a case in which the cell concentration is excessively high, the number of noise components mixed in the spectral data increases. In a case in which the soft sensor 20 is constructed by machine learning using spectral data including a large amount of noise component as the training data, the accuracy of the learning model is reduced, and over-training occurs. As a result, there is a concern that a soft sensor which can withstand actual use will not be constructed. The spectral data is acquired for a cell suspension in which cell concentration has been adjusted to 300 × 10⁶ cells/mL or less, which makes it possible to suppress the amount of noise component mixed in the spectral data within an allowable range. The spectral data is supplied to the information processing device 10. In addition, the perfusion culture is a method which continuously extracts a culture medium including a product, such as an antibody, from the culture vessel 30 and continuously supplies a fresh culture medium to the culture vessel 30.

The state data output from the soft sensor 20 is data indicating the state of the cell or the culture solution correlated with the spectral data. The state data may be, for example, data related to the amount of antibody included in the culture solution, the amount of antibody decomposition product, the amount of antibody aggregate, or the amount of immature sugar chain having a structure similar to that of the antibody. The antibody is a product produced from the cells that are being cultured. Further, the state data may be data related to the amount of component included in the culture solution. In addition, the "data related to the amount" may be the content of the components in the culture solution or may be the content rate (concentration) of the components. Furthermore, the state data may be data related to the number of living cells in the culture vessel 30. The "data related to the number of living cells" may be the total number of living cells in the culture solution or may be the density of the living cells. It is not easy to monitor the state data in line using actual measurement. The use of the soft sensor 20 makes it possible to acquire the state data in line on the basis of the spectral data which is relatively easy to monitor in line using actual measurement.

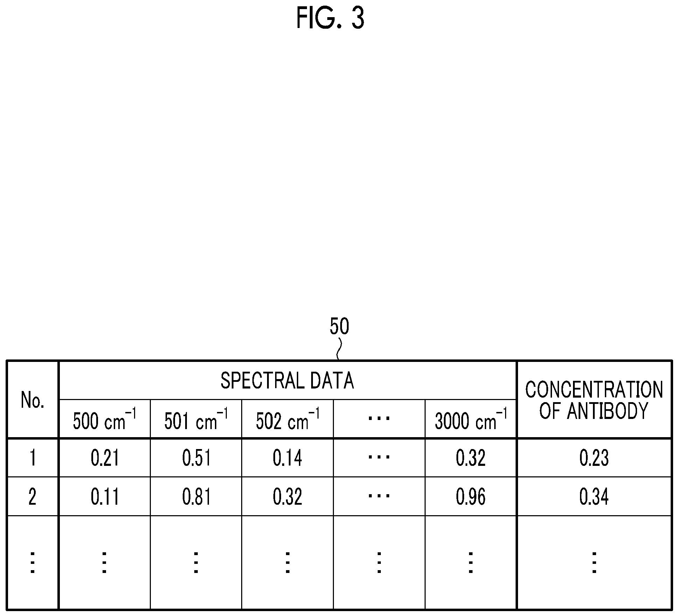

The soft sensor 20 is constructed by machine learning using a plurality of combinations of the spectral data and the state data as the training data. Fig. 3 is a diagram illustrating an example of training data 50. The training data 50 is acquired, for example, in a preliminary test culture. That is, training spectral data is acquired from the cell suspension accommodated in the culture vessel 30 in the test culture. Training state data can be acquired by actual measurement using a sampling method according to the related art in which the cell cultured in the culture vessel 30 or the culture solution is used as an object to be measured in the test culture. For example, in a case in which the concentration of the antibody included in the culture solution is acquired as the training state data, a method, such as high performance liquid chromatography (HPLC), can be used. The test culture is performed for a predetermined period, and the training data is acquired at a plurality of points of time for a test culture period. The training spectral data and the training state data at each point of time are associated with each other.

Here, the analyzer 41 outputs, as the spectral data, the spectral intensity value in the range of, for example, a wave number of 500 cm⁻¹ to 3000 cm⁻¹ at an interval of 1 cm⁻¹. Therefore, the number of spectral data items acquired is enormous. In a case in which all of the spectral data is used as the training data, a learning load becomes excessive, and a high-performance processor is required in order to perform machine learning. Further, in some cases, the spectral intensity values of the Raman scattered light constituting the spectral data include a spectral intensity value for a wave number having a low correlation with the state data to be monitored. For example, it is considered that the spectral intensity value for a specific wave number of the Raman scattered light has a low correlation with the concentration of the antibody. In a case in which the soft sensor 20 is constructed by machine learning using spectral data including the spectral intensity value of the wave number having a low correlation with the state data to be monitored as the training data, there is a concern that the accuracy of the output value of the soft sensor 20 will be reduced.

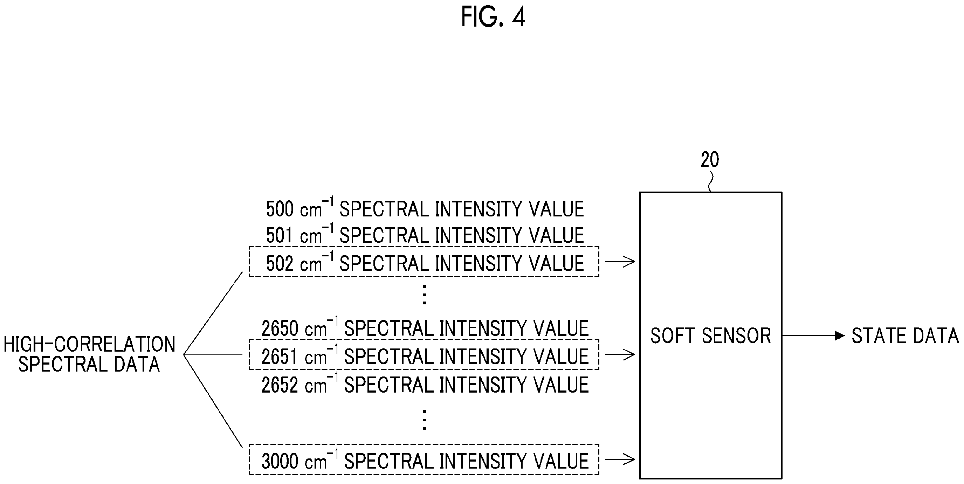

Therefore, in this embodiment, as the preprocessing on the spectral data, among the spectral data output from the analyzer 41, the spectral intensity value of the wave number having a relatively high correlation with the state data to be monitored is specified as the high-correlation spectral data. Then, in a learning phase which is a stage of constructing the soft sensor 20 using machine learning, the soft sensor 20 is constructed by machine learning using a plurality of combinations of the high-correlation spectral data and the state data as the training data. On the other hand, in an operation phase in which the constructed soft sensor 20 is operated to acquire state data for the cells that are being cultured or the culture solution, as illustrated in Fig. 4, among the spectral data acquired for the cell suspension including the cells that are being cultured, high-correlation spectral data having a relatively high correlation with the state data to be monitored is input to the soft sensor 20, and the state data output from the soft sensor 20 is acquired. The information processing device 10 constructs the soft sensor 20 and acquires the state data using the soft sensor 20.

Fig. 5 is a diagram illustrating an example of a hardware configuration of the information processing device 10. The information processing device 10 includes a central processing unit (CPU) 101, a memory 102 as a temporary storage area, and a non-volatile storage unit 103. Further, the information processing device 10 includes a display unit 104, such as a liquid crystal display, an input unit 105, such as a keyboard or a mouse, a network interface (I/F) 106 connected to a network, and an external I/F 107 to which the analyzer 41 is connected. The CPU 101, the memory 102, the storage unit 103, the display unit 104, the input unit 105, the network I/F 106, and the external I/F 107 are connected to a bus 108.

The storage unit 103 is implemented by a storage medium, such as a hard disk drive (HDD), a solid state drive (SSD), or a flash memory. The training data 50, an estimation model 60, a soft sensor construction program 70, and an estimation program 80 are stored in the storage unit 103. As illustrated in Fig. 3, the training data 50 is a plurality of combinations of the spectral data and the state data and is acquired in the test culture.

Fig. 6 is a diagram illustrating an example of the structure of the estimation model 60. The estimation model 60 is a neural network including an input layer, a plurality of middle layers, and an output layer. The spectral intensity value for each wave number of the Raman scattered light, that is, the spectral data is input to the input layer of the estimation model 60. State data corresponding to the spectral data input to the input layer is output from the output layer of the estimation model 60.

In the learning phase, the CPU 101 reads the soft sensor construction program 70 from the storage unit 103, expands the soft sensor construction program 70 in the memory 102, and executes the soft sensor construction program 70. In the operation phase, the CPU 101 reads the estimation program 80 from the storage unit 103, expands the estimation program 80 in the memory 102, and executes the estimation program 80. In addition, a server computer or the like is given as an example of the information processing device 10. The CPU 101 is an example of a processor according to the technology of the present disclosure.

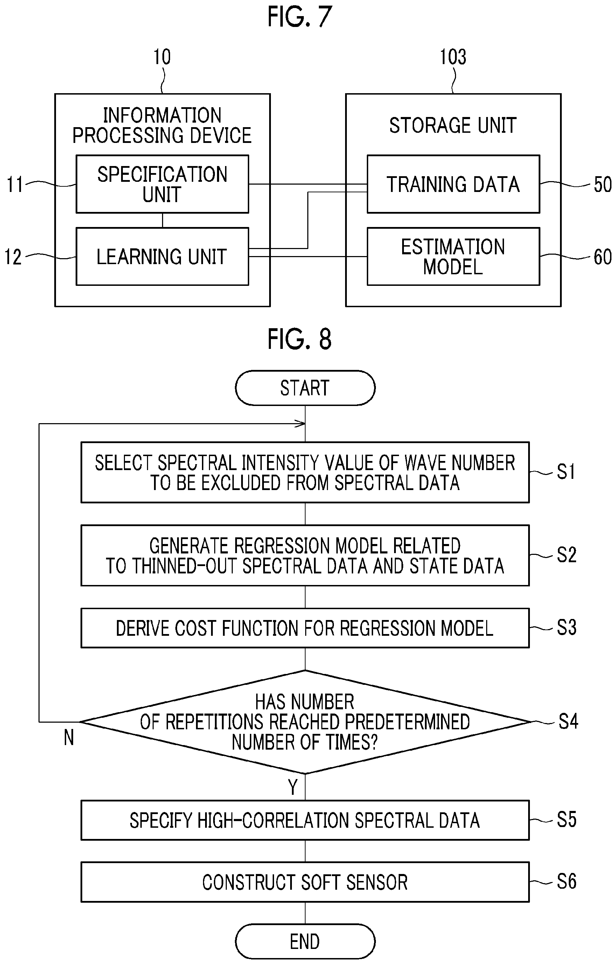

Fig. 7 is an example of a functional block diagram illustrating an example of a functional configuration of the information processing device 10 in the learning phase. In the learning phase, the information processing device 10 is configured to include a specification unit 11 and a learning unit 12. It is assumed that the training data 50 and the estimation model 60 are stored in the storage unit 103.

The specification unit 11 performs regression analysis on the training data 50, using lasso regression which is an example of the sparse modeling, to specify the spectral intensity value of the wave number having a relatively high correlation with the state data as the high-correlation spectral data among the spectral data included in the training data 50. Specifically, the specification unit 11 performs the following process. The specification unit 11 performs a process of thinning out the spectral intensity value of the wave number, which has been determined randomly, on the spectral data included in the training data 50 and generates a regression model (regression expression) indicating the relationship between the thinned-out spectral data and the corresponding state data. The specification unit 11 derives a cost function obtained by adding a penalty term to a root mean squared error (RMSE) for the generated regression model. The specification unit 11 repeatedly performs each of the above-mentioned processes a predetermined number of times to generate the regression model for each of a plurality of spectral data items having different wave numbers to be thinned out and to derive the above-mentioned cost function for each regression model. The specification unit 11 specifies the smallest number of spectral intensity values that can minimize the above-mentioned cost function as the high-correlation spectral data in a predetermined number of repeated calculation operations. For example, in a case in which this preprocessing was applied to glutamine, the following results were obtained for the condition of the number of spectral data items: 2500 and the cost function: 0.23 before the preprocessing, and the number of spectral data items: 20 and the cost function: 0.015 after the preprocessing.

The learning unit 12 trains the estimation model 60 with machine learning using a combination of the high-correlation spectral data specified by the specification unit 11 and the corresponding state data in the training data 50 as teacher data. Therefore, the soft sensor 20 that receives the high-correlation spectral data as an input and outputs the state data is constructed.

The learning unit 12 trains the estimation model 60 using the training data 50 according to a back-propagation method which is an example of machine learning. Specifically, the learning unit 12 extracts the high-correlation spectral data specified by the specification unit 11 from the training spectral data included in the training data 50. The learning unit 12 inputs the extracted high-correlation spectral data to the estimation model 60 and acquires the state data output from the estimation model 60. The learning unit 12 trains the estimation model 60 such that a difference between a score (for example, a score indicating the concentration of the antibody) indicated by the acquired state data and a score indicated by the training state data corresponding to the high-correlation spectral data included in the training data 50 is minimized. The learning unit 12 performs a process of training the estimation model 60 using a combination of all or some of the high-correlation spectral data and the state data included in the training data 50. Further, in addition to the back-propagation method, random forest, linear regression, non-linear regression (Sapport vector machine (SVM) and Basian regression), logistic regression, and the like are given as examples of the machine learning method. However, the back-propagation method is preferable.

Fig. 8 is a flowchart illustrating an example of a flow of the soft sensor construction process performed by the execution of the soft sensor construction program 70 by the CPU 101 in the learning phase. The soft sensor construction program 70 is executed, for example, in a case in which an instruction to perform the soft sensor construction process is input by the user through the input unit 105.

In Step S1, the specification unit 11 randomly selects the spectral intensity value of the wave number to be excluded from the spectral data included in the training data 50 stored in the storage unit 103. That is, the specification unit 11 performs a process of thinning out the spectral intensity values for some wave numbers among the spectral intensity values acquired at a wave number interval of 1 cm⁻¹. The number of wave numbers to be excluded may be predetermined or randomly determined.

In Step S2, the specification unit 11 generates a regression model (regression expression) indicating the relationship between spectral data (that is, thinned-out spectral data) composed of the spectral intensity values of wave numbers other than the wave numbers to be excluded, which have been selected in Step S1, and the corresponding state data. Specifically, a regression model that uses the thinned-out spectral data as an explanatory variable and uses the corresponding state data as an objective variable is estimated by a statistical method. The regression model may be a linear model or a non-linear model.

In Step S3, the specification unit 11 derives a cost function for the regression model generated in Step S2. The cost function is used as an index value indicating the accuracy of the regression model.

In Step S4, the specification unit 11 determines whether or not the number of repetitions of the processes from Step S1 to Step S3 has reached a predetermined number of times. The specification unit 11 repeatedly performs the processes from Step S1 to Step S3 until the number of repetitions reaches a predetermined number of times. Therefore, the regression model is generated for each of a plurality of thinned-out spectral data items having different wave numbers to be excluded, and the cost function is derived for each of the generated regression models.

In Step S5, the specification unit 11 specifies the thinned-out spectral data used to generate the regression model having the minimum cost function as the high-correlation spectral data. The spectral data used to generate the regression model having the minimum cost function is composed of the spectral intensity value of the wave number having a relatively high correlation with the state data. In this way, the specification unit 11 specifies the spectral data composed of the spectral intensity values of the wave numbers having a relatively high correlation with the state data as the high-correlation spectral data, using the regression analysis.

In Step S6, the learning unit 12 extracts the high-correlation spectral data specified in Step S5 from the spectral data included in the training data 50 stored in the storage unit 103 and trains the estimation model 60 with machine learning using a plurality of combinations of the extracted high-correlation spectral data and the corresponding state data as the teacher data. Specifically, the learning unit 12 inputs the high-correlation spectral data specified in Step S5 to the estimation model 60 and trains the estimation model 60 such that the difference between the score indicated by the state data output from the estimation model 60 and the score indicated by the training state data corresponding to the high-correlation spectral data included in the training data 50 is minimized. In this way, the soft sensor 20 is constructed.

The soft sensor 20 is constructed for each type of state data to be monitored. For example, in a case in which data related to the amount of antibody included in the culture solution is output as the state data to the soft sensor 20, the spectral intensity value of the wave number having a high correlation with the amount of antibody in the spectral data is specified as the high-correlation spectral data. Then, the soft sensor 20 that outputs an estimated value of the amount of antibody on the basis of the high-correlation spectral data is constructed by machine learning using a plurality of combinations of the specified high-correlation spectral data and the state data related to the amount of antibody acquired by actual measurement as the training data. Meanwhile, in a case in which data related to the number of cells is output as the state data to the soft sensor 20, the spectral intensity value of the wave number having a high correlation with the number of cells in the spectral data is specified as the high-correlation spectral data. Then, the soft sensor 20 that outputs an estimated value of the number of cells on the basis of the high-correlation spectral data is constructed by machine learning using a plurality of combinations of the specified high-correlation spectral data and the state data related to the number of cells acquired by actual measurement as the training data.

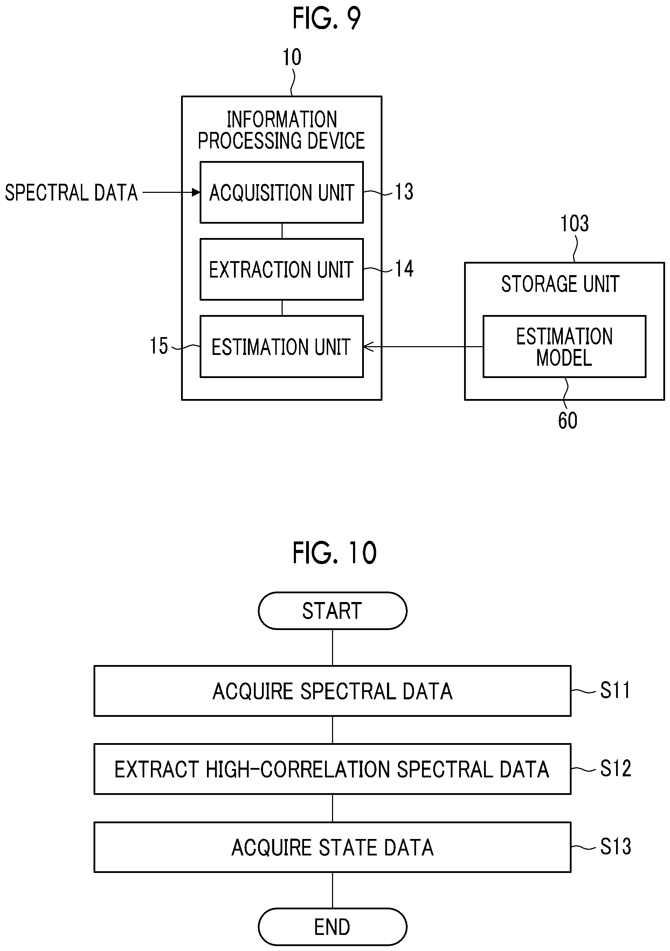

Fig. 9 is an example of a functional block diagram illustrating an example of a functional configuration of the information processing device 10 in the operation phase. In the operation phase, the information processing device 10 is configured to include an acquisition unit 13, an extraction unit 14, and an estimation unit 15. It is assumed that the storage unit 103 stores the trained estimation model 60 functioning as the soft sensor 20.

The method for estimating the culture state according to the embodiment of the technology of the present disclosure is applied to, for example, a case in which the state of the cells, which are being cultured, or the culture solution accommodated in the culture vessel is estimated. As illustrated in Fig. 2, the probe 40 and the analyzer 41 acquire spectral data for the cell suspension 31 including the cells which are being cultured in the culture vessel 30.

The acquisition unit 13 acquires the spectral data output from the analyzer 41. The extraction unit 14 extracts the high-correlation spectral data specified by the specification unit 11, that is, the spectral intensity value of the wave number having a relatively high correlation with the state data to be monitored, among the spectral data acquired by the acquisition unit 13.

The estimation unit 15 reads the trained estimation model 60 functioning as the soft sensor 20 from the storage unit 103, inputs the high-correlation spectral data extracted by the extraction unit 14 to the estimation model 60, and acquires the state data output from the estimation model 60. The estimation unit 15 may perform control to display the acquired state data on the display unit 104. Further, the estimation unit 15 may store the acquired state data in the storage unit 103.

Fig. 10 is a flowchart illustrating an example of a flow of an estimation process performed by the execution of the estimation program 80 by the CPU 101 in the operation phase. The estimation program 80 is executed, for example, in a case in which an instruction to perform the estimation process is input by the user through the input unit 105.

In Step S11, the acquisition unit 13 acquires the spectral data output from the analyzer 41. In Step S12, the extraction unit 14 extracts the high-correlation spectral data specified by the specification unit 11, that is, the spectral intensity value of the wave number having a relatively high correlation with the state data to be monitored, among the spectral data acquired by the acquisition unit 13. In Step S13, the estimation unit 15 reads the trained estimation model 60 functioning as the soft sensor 20 from the storage unit 103, inputs the high-correlation spectral data extracted in Step S12 to the read estimation model 60, and acquires the state data output from the estimation model 60. The estimation unit 15 performs control to display the acquired state data on the display unit 104.

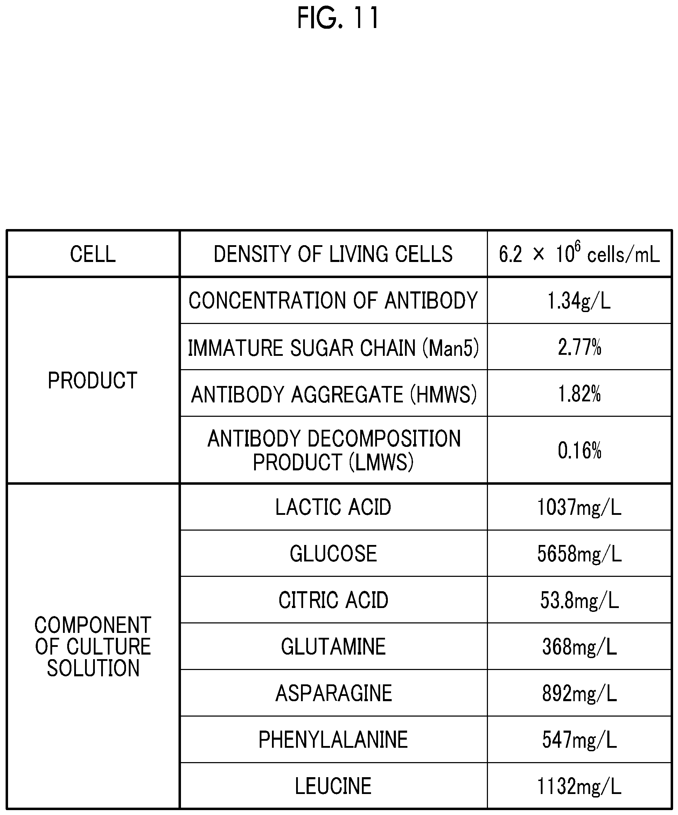

Fig. 11 is a diagram illustrating an example of the state data acquired by the soft sensor 20. The use of the soft sensor 20 makes it possible to estimate, for example, the density of living cells as an item related to the cells. In addition, the use of the soft sensor 20 makes it possible to estimate, for example, the concentration of antibodies, the concentration of immature sugar chains having a structure similar to that of the antibody, the concentration of antibody aggregates, and the concentration of antibody decomposition products as items related to the product. Further, the use of the soft sensor 20 makes it possible to estimate, for example, the concentration of lactic acid, the concentration of glucose, the concentration of citric acid, the concentration of glutamine, the concentration of asparagine, the concentration of phenylalanine, and the concentration of leucine as items related to the components included in the culture solution.

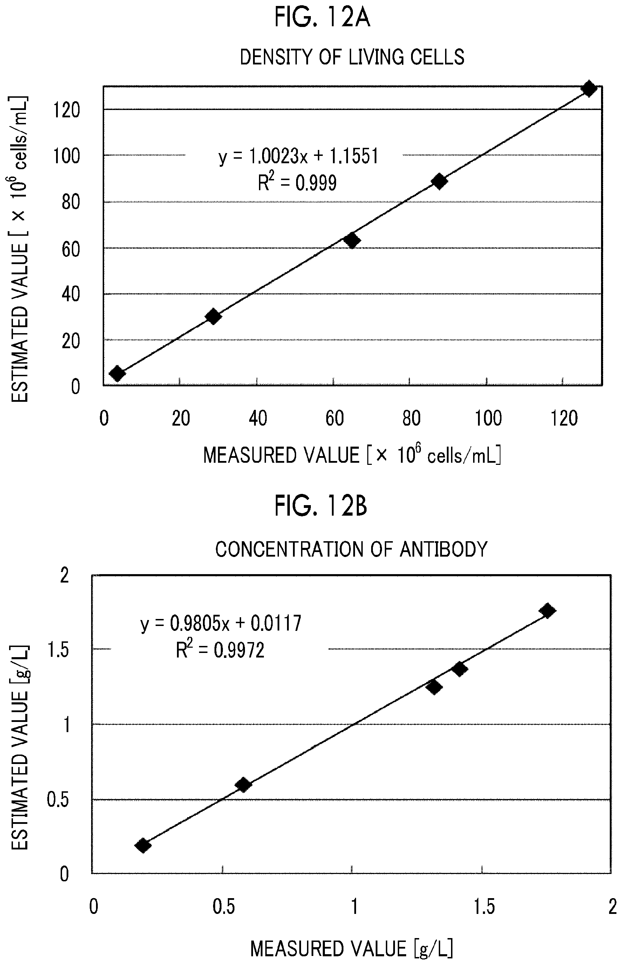

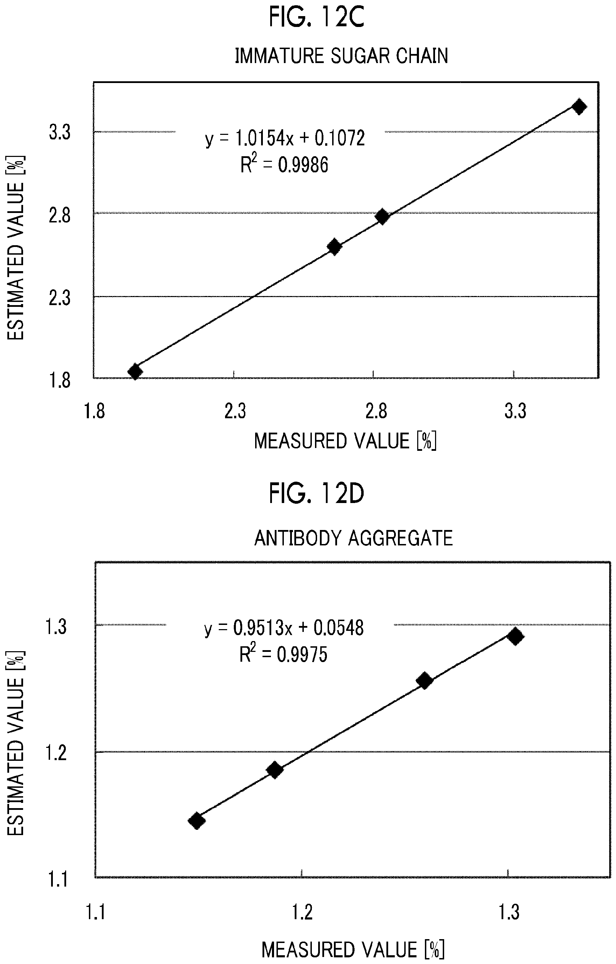

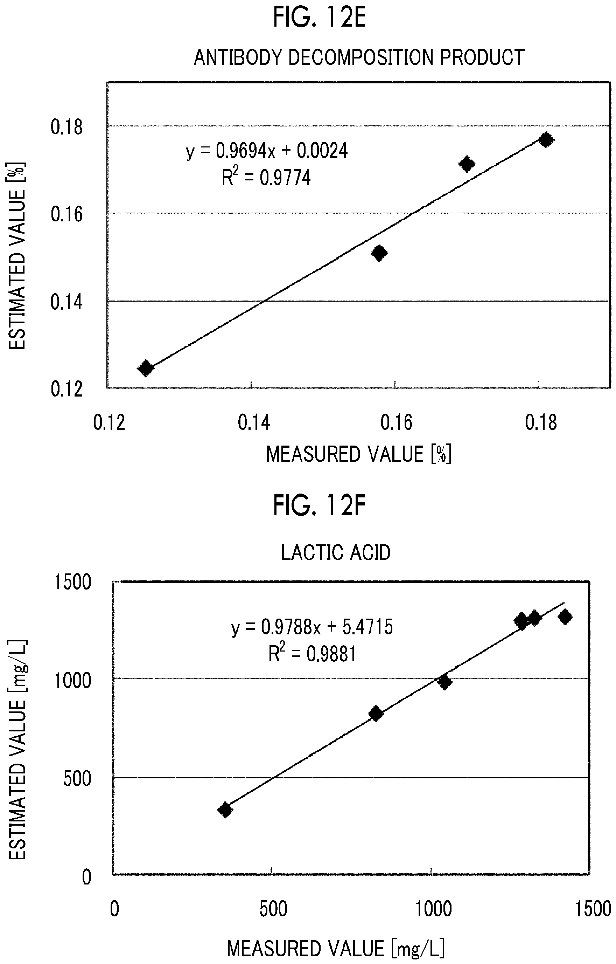

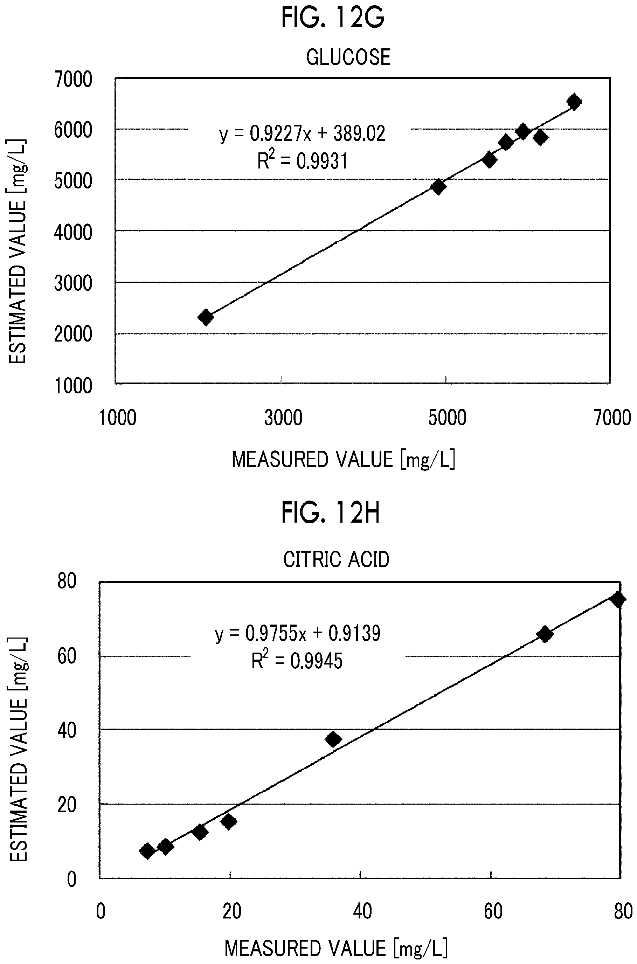

Figs. 12Ato 12L are graphs illustrating the relationships between the estimated values of each of the items illustrated in Fig. 11, which are the state data output from the soft sensor 20, and the measured values acquired by sampling. It was confirmed that, in any of the items, the value of a determination coefficient R² indicating the degree of deviation of the estimated value acquired by the soft sensor 20 from the measured value was equal to or greater than 0.95 and that the accuracy of the output value of the soft sensor 20 was very high.

Fig. 13 is a graph illustrating the relationship between the estimated value of the concentration of glutamine included in the culture solution and the measured value in a case in which the estimated value is acquired by the soft sensor 20 and in a case in which the estimated value is acquired by a multivariate analysis method using PLS according to the related art. Figs. 14A and 14B are graphs illustrating the determination coefficient R² indicating the degree of deviation of the estimated value of the concentration of each component included in the culture solution from the measured value in a case in which the estimated value is acquired by the soft sensor 20 and in a case in which the estimated value is acquired by the multivariate analysis method using PLS according to the related art. In the case of using the soft sensor 20, the determination coefficient R² was acquired for each of a case in which the preprocessing for specifying the high-correlation spectral data was performed and a case in which the preprocessing was not performed. Table 1 shows the number of wave numbers (the number of explanatory variables) used in concentration estimation in each method (the soft sensor (with preprocessing), the soft sensor (without preprocessing), and PLS). In PSL, in a case in which the entire wave number band (2500) of the Raman scattered light is included in the object to be analyzed, accuracy is significantly reduced. Therefore, 1000 wave numbers from 500 cm⁻¹ to 1500 cm⁻¹ having peaks caused by, for example, C-H bond or C-O bond in each of the above-mentioned components to be subjected to concentration estimation were the objects to be analyzed. The spectral data used in concentration estimation in each method is spectral data obtained by the same culture experiment. In addition, it was difficult to acquire the estimated values of the concentration of lactic acid and the concentration of citric acid with the multivariate analysis method using PLS. It was confirmed that, for all of the components included in the culture solution, the accuracy of the estimated values acquired by the soft sensor 20 was higher than the accuracy of the estimated values acquired by the multivariate analysis method using PLS according to the related art as illustrated in Figs. 13, 14A, and 14B. Further, it was confirmed that, in a case in which the soft sensor 20 was used, the preprocessing for specifying the high-correlation spectral data was performed to further increase the accuracy of the estimated values. In this embodiment, among the spectral intensity values (the number of data items: 2500) for each wave number or wavelength included in the spectral data, 11 or more and 334 or less spectral intensity values used as the training data in a case in which the soft sensor 20 was constructed were selected by the preprocessing.

**[Table 1]**

| | PLS | Soft sensor without preprocessing | Soft sensor with preprocessing |
|---|---|---|---|
| Glutamine | 1000 | 2500 | 20 |
| Glucose | 1000 | 2500 | 43 |
| Leucine | 1000 | 2500 | 80 |
| Phenylalanine | 1000 | 2500 | 109 |
| Asparagine | 1000 | 2500 | 334 |
| Lactic acid | 1000 | 2500 | 25 |
| Citric acid | 1000 | 2500 | 38 |
| Density of living cells | 1000 | 2500 | 73 |
| Concentration of antibody | 1000 | 2500 | 30 |
| Immature sugar chain | 1000 | 2500 | 222 |
| Antibody aggregate | 1000 | 2500 | 11 |
| Antibody decomposition product | 1000 | 2500 | 191 |

As described above, according to the method for estimating the culture state of the embodiment of the technology of the present disclosure, the culture state is estimated by inputting the spectral data acquired for the cell suspension including the cells, which are being cultured, to the soft sensor and acquiring the state data output from the soft sensor. Since the in-line monitoring of the spectral data by actual measurement is relatively easy, it is possible to estimate the culture state in line. In addition, since the estimation result of the culture state can be acquired immediately, it is possible to immediately respond to a case in which any abnormality occurs for a culture period.

Further, the soft sensor is constructed by machine learning using a plurality of combinations of the spectral data and the state data acquired in the actual culture environment as the training data. Therefore, even in the actual culture environment in which the density of the cells in the culture vessel is high and the content of the object to be monitored is low, it is possible to estimate the culture state with higher accuracy than that in a case in which the state of the cells is estimated using the multivariate analysis method such as PLS.

Further, among the spectral data output from the analyzer 41, the high-correlation spectral data composed of the spectral intensity value of the wave number having a relatively high correlation with the state data to be monitored is used as the training data. Therefore, a learning load can be less than that in a case in which all of the spectral data output from the analyzer 41 is used as the training data. In addition, it is possible to improve the accuracy of the output value of the soft sensor 20. For the state data, the value of the determination coefficient R² indicating the degree of deviation between the estimated value acquired by the soft sensor and the measured value acquired by sampling is preferably equal to or greater than 0.8, more preferably equal to or greater than 0.9, and most preferably equal to or greater than 0.95.

Further, according to the method for estimating the culture state of the embodiment of the technology of the present disclosure, it is possible to acquire, as the state data, the estimated values of the amount of antibody included in the culture solution, the amount of antibody decomposition product, the amount of antibody aggregates, the amount of immature sugar chains having a structure similar to that of the antibody. The antibody decomposition product is formed by the decomposition of the antibody by a degrading enzyme generated during culture. The antibody aggregates are likely to be formed, for example, in a case in which the concentration of the antibody produced by the cells is excessively high or in a case in which stress, such as heat, is applied. The immature sugar chains having a structure similar to that of the antibody are likely to be formed, for example, in a case in which the amount of waste products in the culture solution increases or in a case in which oxygen concentration in the culture solution is insufficient. It is preferable to minimize the amounts of antibody decomposition product, antibody aggregates, and immature sugar chains having a structure similar to that of the antibody which are produced during the culture period. Therefore, it is effective to acquire the estimated value of the amount of antibody decomposition product, the amount of antibody aggregates, or the amount of immature sugar chains having a structure similar to that of the antibody as the state data in order to maintain a good culture state.

Further, according to the method for estimating the culture state of the embodiment of the technology of the present disclosure, it is possible to acquire the estimated value of the amount of component included in the culture solution as the state data. The culture solution includes, for example, nutrients necessary to culture cells. It is preferable that necessary nutrients are included in the culture solution without excess or deficiency for the culture period. Therefore, it is effective to acquire the estimated value of the amount of component included in the culture solution as the state data in order to maintain a good culture state.

Further, according to the method for estimating the culture state of the embodiment of the technology of the present disclosure, it is possible to acquire the estimated value of the number of cells cultured in the culture vessel 30 as the state data. In a case in which the number of cells cultured in the culture vessel 30 is excessively large, oxygen and nutrients supplied to the cells are insufficient. As a result, there is a concern that the survival rate of the cells will be reduced. Meanwhile, in cell culture for producing antibodies, in a case in which the number of cells cultured in the culture vessel 30 is excessively small, the efficiency of producing antibodies is reduced. Therefore, it is effective to acquire the estimated value of the number of cells cultured in the culture vessel 30 as the state data in order to maintain a good culture state.

In this embodiment, the aspect in which the spectrum of Raman scattered light is used as the spectral data has been described as an example. However, this embodiment is not limited to this aspect. For example, the absorption spectrum of infrared rays emitted to the cell suspension may be used as the spectral data. In addition, a nuclear magnetic resonance spectrum may be used as the spectral data.

Further, in this embodiment, a case in which preprocessing is performed on the spectral data and the soft sensor is constructed by machine learning using a plurality of combinations of the processed data obtained by the preprocessing and the state data as the training data has been described as an example. However, in a case in which a reduction in the accuracy of the estimation model caused by the learning load and over-training does not cause a problem, spectral data that has not been subjected to the preprocessing may be used as the training data.

Furthermore, in this embodiment, a process for specifying the high-correlation spectral data having a relatively high correlation with the state data among the spectral data has been described as an example of the preprocessing. However, the present disclosure is not limited thereto. For example, a process that excludes the spectral intensity value of a predetermined wave number among the spectral data acquired by the analyzer 41 from the training data may be performed as the preprocessing. In addition, a process that groups the spectral data acquired by the analyzer 41 such that wave numbers close to each other belong to the same wave number group and calculates, for example, the average value, standard deviation, median value, maximum value, and minimum value of the intensity of scattered light for each wave number group may be performed as the preprocessing. In this case, the spectral intensity value for each wave number group is used as the training data. Further, a process that reduces the number of dimensions for the training data composed of a plurality of combinations of the spectral data indicating intensity for each wave number or each wavelength and the state data may be performed as the preprocessing.

Furthermore, in the above-described embodiment, for example, the following various processors can be used as a hardware structure of processing units performing various processes such as the specification unit 11, the learning unit 12, the acquisition unit 13, the extraction unit 14, and the estimation unit 15. The various processors include, for example, a CPU which is a general-purpose processor executing software (program) to function as various processing units, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or a combination of two or more processors of the same type or different types (for example, a combination of a plurality of FPGAs or a combination of a CPU and an FPGA). In addition, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system on chip (SoC). As such, various processing units are configured by using one or more of the various processors as a hardware structure.

In addition, specifically, an electric circuit (circuitry) obtained by combining circuit elements, such as semiconductor elements, can be used as the hardware structure of the various processors.

Further, in the above-described embodiment, the aspect in which the soft sensor construction program 70 and the estimation program 80 are stored (installed) in the storage unit 103 in advance has been described. However, the present disclosure is not limited thereto. The soft sensor construction program 70 and the estimation program 80 may be recorded on a recording medium, such as a compact disc read only memory (CD-ROM), a digital versatile disc read only memory (DVD-ROM), or a universal serial bus (USB) memory, and then provided. Further, the soft sensor construction program 70 and the estimation program 80 may be downloaded from an external device through a network.

In addition, the disclosure of JP2020-075480 filed on April 21, 2020 is incorporated herein by reference in its entirety. Further, all documents, patent applications, and technical standards described in the specification are incorporated herein by references to the same extent as the incorporation of the individual documents, patent applications, and technical standards by references are described specifically and individually.

## Claims

1. A method for estimating a culture state, the method comprising:
acquiring spectral data indicating an intensity of electromagnetic waves, which have been emitted to a cell suspension that includes a cell and a culture solution and has a cell concentration of 35 × 10⁶ cells/mL or more and have been subjected to an action of the cell suspension, for each wave number or wavelength;
constructing a soft sensor, which receives the spectral data as an input and outputs state data indicating a state of the cell or the culture solution, with machine learning using a plurality of combinations of the spectral data and the state data as training data; and
inputting the spectral data acquired for a cell suspension including a cell which is being cultured to the soft sensor and acquiring the state data output from the soft sensor.

2. A method for estimating a culture state, the method comprising:
acquiring spectral data indicating an intensity of electromagnetic waves, which have been emitted to a cell suspension including a cell and a culture solution and have been subjected to an action of the cell suspension, for each wave number or wavelength;
performing preprocessing on the spectral data;
constructing a soft sensor, which receives processed data obtained by the preprocessing as an input and outputs state data indicating a state of the cell or the culture solution, with machine learning using a plurality of combinations of the processed data and the state data as training data; and
inputting the processed data for the spectral data acquired for a cell suspension including a cell which is being cultured to the soft sensor and acquiring the state data output from the soft sensor.

3. The estimation method according to claim 2,
wherein the preprocessing includes a process of selecting, from spectral intensity values for each wave number or wavelength included in the spectral data, a spectral intensity value used as the training data.

4. The estimation method according to claim 3,
wherein, among the spectral intensity value for each wave number or wavelength included in the spectral data, the number of spectral intensity value selected to be used as the training data is equal to or greater than 5 and less than 1000.

5. The estimation method according to claim 3 or 4,
wherein the selection is performed by sparse modeling.

6. The estimation method according to any one of claims 2 to 5,
wherein the preprocessing includes specifying high-correlation spectral data having a relatively high correlation with the state data among the spectral data as the processed data.

7. The estimation method according to any one of claims 1 to 6,
wherein the spectral data is data indicating a spectrum of scattered light of light emitted to the cell suspension.

8. The estimation method according to any one of claims 1 to 7,
wherein the state data is data related to an amount of antibody included in the culture solution, an amount of decomposition product of the antibody, an amount of aggregate of the antibody, or an amount of immature sugar chain having a structure similar to that of the antibody.

9. The estimation method according to any one of claims 1 to 7,
wherein the state data is data related to an amount of component included in the culture solution.

10. The estimation method according to any one of claims 1 to 7,
wherein the state data is data related to the number of the cells.

11. An information processing device comprising at least one processor,
wherein the processor performs preprocessing on spectral data indicating an intensity of electromagnetic waves, which have been emitted to a cell suspension including a cell and a culture solution and have been subjected to an action of the cell suspension, for each wave number or wavelength, and
constructs a soft sensor, which receives processed data obtained by the preprocessing as an input and outputs state data indicating a state of the cell or the culture solution, with machine learning using a plurality of combinations of the processed data and the state data as training data.

12. A program that causes a computer to execute a process comprising:
performing preprocessing on spectral data indicating an intensity of electromagnetic waves, which have been emitted to a cell suspension including a cell and a culture solution and have been subjected to an action of the cell suspension, for each wave number or wavelength; and
constructing a soft sensor, which receives processed data obtained by the preprocessing as an input and outputs state data indicating a state of the cell or the culture solution, with machine learning using a plurality of combinations of the processed data and the state data as training data.
